## Europäisches Patentamt
## European Patent Office
## Office européen des brevets

(19)

(11) Veröffentlichungsnummer: **0 111 786**
**B1**

(12)

# EUROPÄISCHE PATENTSCHRIFT

(45) Veröffentlichungstag der Patentschrift:
**03.09.86**

(51) Int. Cl.⁴: **A 61 M 25/00**

(21) Anmeldenummer: **83111985.4**

(22) Anmeldetag: **30.11.83**

(54) Venenkatheter.

(30) Priorität: **14.12.82 DE 8235102 U**

(43) Veröffentlichungstag der Anmeldung:
**27.06.84 Patentblatt 84/26**

(45) Bekanntmachung des Hinweises auf die Patenterteilung:
**03.09.86 Patentblatt 86/36**

(84) Benannte Vertragsstaaten:
**AT BE CH DE FR GB IT LI NL SE**

(56) Entgegenhaltungen:
**EP-A-0 021 446**
**EP-A-0 054 728**
**EP-A-0 064 212**
**FR-A-2 364 665**
**GB-A-2 033 236**
**US-A-3 603 311**
**US-A-3 610 239**
**US-A-3 677 243**

(73) Patentinhaber: **Nessler, Reiner, Dr.,**
**Paracelsiusstrasse 24, D-3320 Salzgitter- Bad (DE)**

(72) Erfinder: **Nessler, Reiner, Dr., Paracelsiusstrasse**
**24, D-3320 Salzgitter- Bad (DE)**

(74) Vertreter: **Gramm, Werner, Prof. Dipl.- Ing.,**
**Patentanwälte Gramm + Lins Theodor- Heuss-**
**Strasse 2, D-3300 Braunschweig (DE)**

## Beschreibung

Die Erfindung betrifft einen Venenkatheter, bestehend aus einer Kunststoffkanüle, deren eines Ende als Kupplungsstück größeren Durchmessers ausgebildet ist, in das ein Einschubkonus einer Schutztüte lösbar gesteckt ist, die einen flexiblen, mit einer herausziehbaren Sonde bestückten Katheterschlauch umhüllt, dessen Außendurchmesser größer ist als der lichte Innendurchmesser der Kunststoffkanüle, die in Längsrichtung verlaufende Reihen von Querschnittsschwächungen oder Längstrennstellen aufweist.

Eine derartige Ausführungsform läßt sich der EP-A-0 064 212 entnehmen. Die in Längsrichtung verlaufenden Reihen von Querschnittsschwächungen oder Längstrennstellen erstrecken sich nur über eine Teillänge der Kunststoffkanüle, deren übrige Teillänge mit den Bereichen versehen ist, die eine Aufweitung der Kunststoffkanüle zulassen. Das Kupplungsstück weist weder Querschnittsschwächungen noch eine Längstrennstelle auf. Beim Einführen und Vorschieben des Katheterschlauches führt dessen in Vorschubrichtung liegendes vorderes Ende zu einer Aufspreizung bzw. Aufweitung der Kunststoffkanüle, damit sich deren Reibungswiderstand beim Zurückziehen der Kunststoffkanüle über den Katheterschlauch verringert.

Die Aufspreizung bzw. Dehnung der Kunststoffkanüle erfolgt also durch Verdrängungsarbeit, die durch das vordere Ende des Katheterschlauches aufgebracht werden muß. Dadurch besteht die Gefahr einer Katheterbeschädigung. Nachteilig ist ferner, daß die Kunststoffkanüle auch noch dann als einteiliges Führungselement vorliegt, wenn der Katheterschlauch bereits in ein Blutgefäß eingeführt worden ist. Dadurch ist es erforderlich, die Kunststoffkanüle über den Katheterschlauch zurückzuziehen und zwar immer erst dann, wenn der Katheterschlauch bereits plaziert ist.

Die EP-A-0 021 446 offenbart eine teilbare Kunststoffkanüle mit Kegelansatz. Auch hier dient die Kunststoffkanüle als Hilfsvorrichtung zur Einführung eines flexiblen Katheterschlauches in ein Blutgefäß. Nach der Einführung des Katheterschlauches wird die Kunststoffkanüle aus der Punktionsstelle herausgezogen und dann in Längsrichtung so getrennt, daß sie von dem verbleibenden Katheterschlauch entfernt werden kann. Hierzu faßt man die am Ansatz der Kunststoffkanüle vorgesehene Griffplatte zwischen Daumen und Zeigefinger jeder Hand und zieht die Griffplatten nach der Seite, reißt dabei die Kunststoffkanüle ein und trennt sie bei weiterem seitlichen Ziehen der Länge nach auf. Die Griffplatten und die längsgeteilte Kunststoffkanüle lassen sich dann von dem Katheterschlauch vollständig entfernen. Es ist also erforderlich, die Kunststoffkanüle zuerst aus der Punktionsstelle herauszuziehen, bevor sie getrennt und vom Katheterschlauch entfernt werden kann.

Der Erfindung liegt die Aufgabe zugrunde, den eingangs erläuterten Venenkatheter so zu verbessern, daß er nur ein verringertes Punktionstrauma erfordert und eine vereinfachte Trennung vom Katheterschlauch zuläßt.

Diese Aufgabe wird gemäß der Erfindung dadurch gelöst, daß sich zwei gegenüberliegende Reihen von Querschnittsschwächungen bzw. Längstrennstellen über die volle Länge der Kunststoffkanüle erstrecken und so zwei gleiche Längshälften bilden, deren Verbindungselemente durch eine im Kupplungsstück angeordnete Trenneinrichtung lösbar sind, die durch zwei Kupplungsstück-Hälften gebildet ist, die gegeneinander versetzte Handhabungslappen o.dergl. aufweisen und zwischen sich eine Verschiebefläche bilden, die mit der durch die beiden genannten Reihen definierten Trennfläche zwischen den beiden Längshälften der Kunststoffkanüle einen spitzen Winkel einschließt.

Durch Betätigung der im Kupplungsstück vorgesehenen Trenneinrichtung läßt sich die Kunststoffkanüle entlang ihrer Längsachse in zwei Längshälften zerlegen. Hierzu ist es lediglich erforderlich, die eine Kupplungsstück-Hälfte gegenüber der anderen in Längsrichtung um ein geringes Maß, das kleiner als 1 cm sein kann, zu verschieben. Dies erfolgt durch Beaufschlagung der gegeneinander versetzten Handhabungslappen. Hierdurch wird die eine Kupplungsstück-Hälfte gegenüber der anderen Hälfte etwas hochgezogen und übt dadurch auf die an ihr hängende Längshälfte der Kunststoffkanüle eine Zugkraft aus, so daß die die beiden Längshälften zusammenhaltenden Verbindungselemente mit einer Scherkraft beaufschlagt werden. Der Trenneffekt wird dadurch erleichtert, daß die Trennfläche zwischen den beiden Längshälften unter einem spitzen Winkel in die Verschiebefläche zwischen den beiden Kupplungsstück-Hälften übergeht. Aufgrund dieser Richtungsänderung werden die Verbindungselemente beim Trennvorgang zusätzlich mit einer in radialer Richtung wirkenden Trennkraft beaufschlagt.

Die zur Trennung der Kunststoffkanüle erforderlichen Kräfte werden also nicht durch den Katheterschlauch selbst aufgebracht, so daß Beschädigungen am in Vorschubrichtung vorn liegenden Ende des Katheterschlauches ausgeschlossen sind. Da die zur Trennung der Kunststoffkanüle erforderliche Relativverschiebung zwischen den beiden Kupplungsstück-Hälften unter einem spitzen Winkel zur Längsmittelachse der Kunststoffkanüle erfolgt, läßt sich die Kunststoffkanüle bereits dann in zwei Hälften zerlegen, wenn sie sich noch in der Punktionsstelle befindet. Dadurch ist ein einfaches und weitgehend kraftfreies Vorschieben des Katheterschlauches möglich. Das Punktionstrauma ist gegenüber dem Lösungsvorschlag gemäß der EP-A-0 021 446 verringert, da dort der Innendurchmesser der noch ungeteilten Kunststoffkanüle geringfügig größer sein muß als der Außendurchmesser des hindurchzuschiebenden Katheterschlauches.

Bei der erfindungsgemäßen Lösung ist es vorteilhaft, daß die beiden Längshälften der Kunststoffkanüle über ihre volle Länge voneinander getrennt werden und dementsprechend auch über ihre volle Länge in radialer Richtung gesehen voneinander weggedrückt werden können. Dies erleichtert das spätere Herausziehen der Längshälften aus dem Punktionskanal bzw. der Haut des Patienten und führt dort nicht zu Verletzungen, die z.B. dann nicht zu vermeiden wären, wenn sich nur das innenliegende Ende der Kunststoffkanüle unter der Einwirkung des eingeschobenen Katheterschlauches pilzartig aufspreizen würde.

Bei dem neuen Venenkatheter kann der Katheterschlauch bereits auf die Sonde aufgezogen sein, die den Katheterschlauch an seinem in Vorschubrichtung vorn liegenden Ende zur Verbesserung der Führung etwas überragen kann. Demgegenüber darf bei der sogenannten indirekten Methode die Sonde nicht vollständig bis zum Ende des Katheterschlauches durchgeführt sein. Das vordere Schlauchende umschließt also einen nach vorn offenen Hohlraum, was bei dem Einführen des Katheterschlauches zum Mitnehmen von Gewebe u.dergl. führt. Auch diese Nachteile lassen sich bei der Anwendung des neuen Venenkatheters vermeiden.

Um ein vorzeitiges Auseinanderfallen der Kunststoffkanüle zu verhindern, ist es vorteilhaft, wenn die beiden Kupplungsstück-Hälften über einen trennbaren Steg miteinander verbunden sind.

In der Zeichnung sind einige als Beispiele dienende Ausführungsformen der Erfindung dargestellt. Es zeigen:

Figur 1 in schematischer Darstellung in Draufsicht einen kompletten Venenkatheter;

Figur 2 in vergrößertem Maßstab eine geringfügig abgewandelte Ausführungsform gemäß Figur 1 und

Figur 3 eine Kunststoffkanüle in einer abgewandelten Ausführungsform.

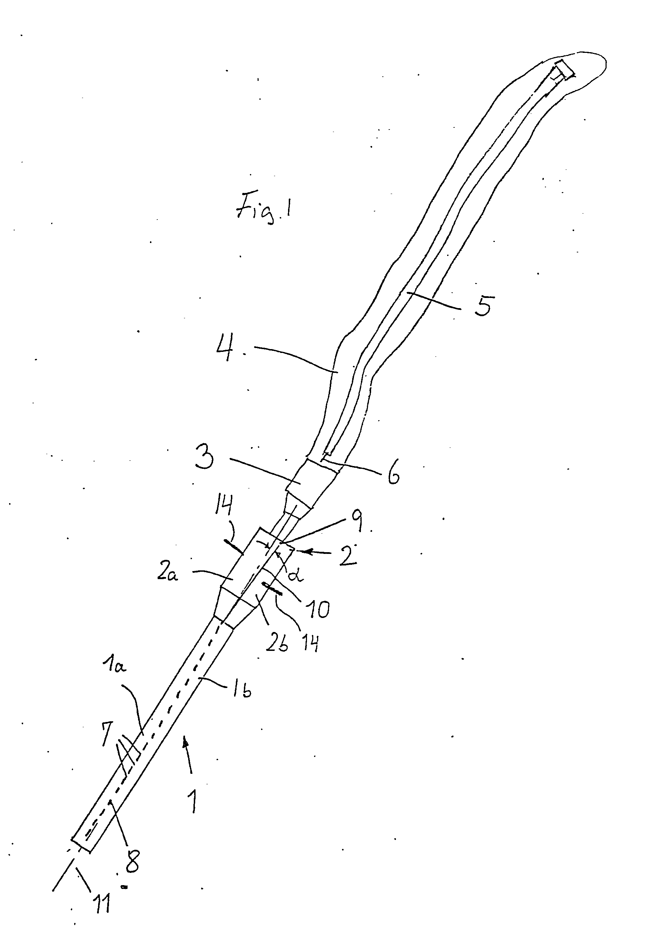

Der Venenkatheter gemäß Figur 1 besteht aus einer Kunststoffkanüle 1, deren eines Ende als Kupplungsstück 2 größeren Durchmessers ausgebildet ist, in das ein Einschubkonus 3 einer Schutztüte 4 lösbar gesteckt ist. Letztere umhüllt einen flexiblen Katheterschlauch 5 mit einer herausziehbaren Sonde 6, die gemäß Darstellung das vordere Schlauchende etwas überragen kann.

Der Außendurchmesser des Katheterschlauches 5 ist größer als der lichte Innendurchmesser der Kunststoffkanüle 1. Letztere besteht aus zwei Längshälften 1a,1b, die über Verbindungselemente zusammenhängen, die durch eine im Kupplungsstück 2 angeordnete Trenneinrichtung lösbar sind.

Bei der in Figur 1 dargestellten Ausführungsform bestehen die genannten Verbindungselemente aus Stegen 7, die durch zwei sich gegenüberliegende, in Längsrichtung verlaufende Reihen 8 von Querschnittsschwächungen gebildet sind. Die genannte Trenneinrichtung wird durch zwei Kupplungsstück-Hälften 2a, 2b gebildet, die relativ zueinander längsverschiebbar und mit jeweils einer Längshälfte 1a, 1b der Kunststoffkanüle 1 verbunden sind.

Die Verschiebefläche 10 zwischen den beiden Kupplungsstück-Hälften 2a, 2b schließt mit der Trennfläche 11 zwischen den beiden Längshälften 1a, 1b der Kunststoffkanüle 1 einen spitzen Winkel ein.

Zur leichteren Bedienung weist bei der Ausführungsform gemäß Figur 1 jede der beiden Kupplungsstück-Hälften 2a, 2b einen Lappen 14 auf, wobei die beiden Lappen 14 gegeneinander versetzt angeordnet sein können.

Die beiden Kupplungsstück-Hälften 2a, 2b sind über einen trennbaren Steg 9 miteinander verbunden.

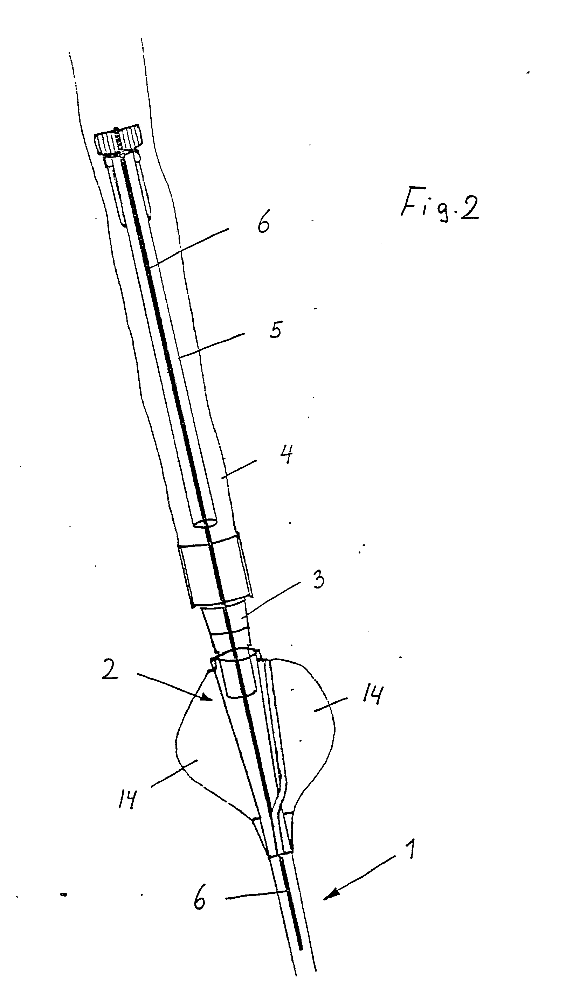

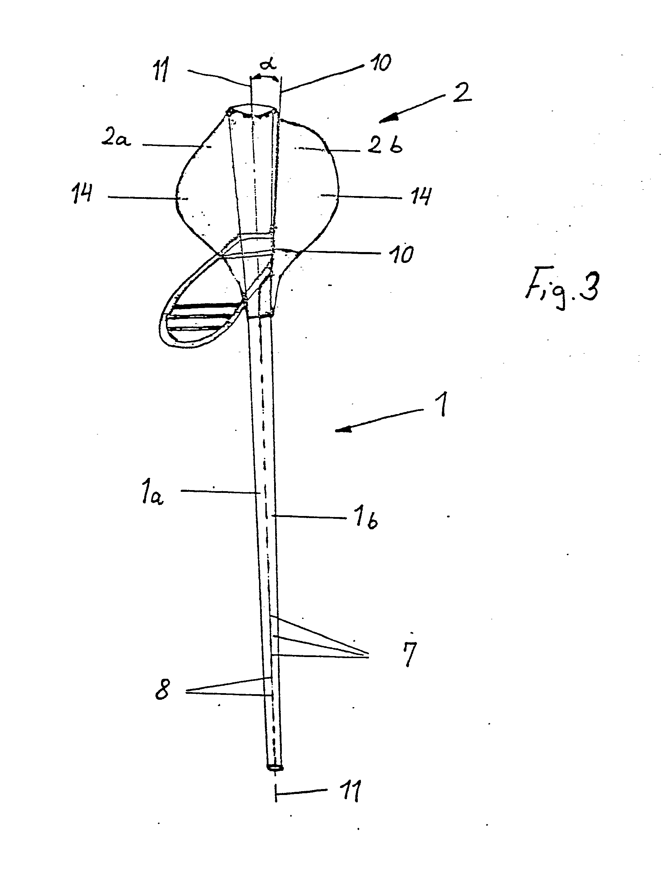

Die in den Figuren 2 und 3 dargestellten Ausführungsformen entsprechen im wesentlichen der der Figur 1, wobei lediglich die Lappen 14 etwas anders angeordnet sind.

**Patentansprüche**

1. Venenkatheter, bestehend aus einer Kunststoffkanüle (1), deren eines Ende als Kupplungsstück (2) größeren Durchmessers ausgebildet ist, in das ein Einschubkonus (3) einer Schutztüte (4) lösbar gesteckt ist, die einen flexiblen, mit einer herausziehbaren Sonde (6) bestückten Katheterschlauch (5) umhüllt, dessen Außendurchmesser größer ist als der lichte Innendurchmesser der Kunststoffkanüle (1), die in Längsrichtung verlaufende Reihen (8) von Querschnittsschwächungen oder Längstrennstellen aufweist, dadurch gekennzeichnet, daß sich zwei gegenüberliegende Reihen (8) von Querschnittsschwächungen bzw. Längstrennstellen über die volle Länge der Kunststoffkanüle (1) erstrecken und so zwei gleiche Längshälften (1a, 1b) bilden, deren Verbindungselemente (7) durch eine im Kupplungsstück (2) angeordnete Trenneinrichtung lösbar sind, die durch zwei Kupplungsstück-Hälften (2a, 2b) gebildet ist, die gegeneinander versetzte Handhabungslappen (14) o.dergl. aufweisen und zwischen sich eine Verschiebefläche (10) bilden, die mit der durch die beiden genannten Reihen (8) definierten Trennfläche (11) zwischen den beiden Längshälften (1a, 1b) der Kunststoffkanüle (1) einen spitzen Winkel ($\alpha$) einschließt.

2. Venenkatheter nach Anspruch 1, dadurch gekennzeichnet, daß die beiden Kupplungsstück-Hälften (2a, 2b) über einen trennbaren Steg (9) miteinander verbunden sind.

## Claims

1. A vein catheter comprising a plastic cannula (1) one end of which being formed as coupling member (2) of greater diameter, into which a plug-in cone (3) of a protective bag (4) is detachably inserted, said protective bag enclosing a flexible catheter tube (5) including a removable probe (6) the outer diameter of said catheter tube being greater than the inner diameter of the plastic cannula (1) which has lines (8) of weakened scores or longitudinal disconnecting points, said lines extending in longitudial direction, <u>characterized</u> in that two opposite lines (8) of weakened scores and longitudinal disconnecting points, respectively extend across the whole length of the plastic cannula (1) and thus form two egual longitudinal halves (1a, 1b), the connecting elements (7) of which being detachable by separating means arranged in the coupling member (2) and formed by two coupling-member halves (2a, 2b) which have staggered handles (14) or the like and form between each other a sliding plane (10) which encloses an acute angle (α) with the joint face (11) defined by said to lines (8) between the two longitudinal halves (1a, 1b) of the plastic cannula (1).

2. The vein catheter accordinq to claim 1, wherein the two coupling-member halves (2a, 2b) are interconnected via a separable web (9).

## Revendications

1. Cathéter intraveineux, composé d'une canule en matière plastique (1), dont une extrémité a la forme d'un organe de raccordement (2) de plus grand diamètre, dans lequel est enfoncé amoviblement un cône d'enfichage (3) d'un sac de protection (4) qui enveloppe un tuyau souple (5) de cathéter muni d'une sonde extractible (6), le diamètre extérieur du tuyau souple étant plus grand que le diamètre intérieur de la canule en matière plastique (1), laquelle présente des rangées (8), divisées longitudinalement, d'affaiblissements de la section ou de points de séparation longitudinale, caractérisé en ce que deux rangées (8) d'affaiblissements de la section ou de points de séparation longitudinale situées l'une en face de l'autre s'étendent sur toute la longueur de la canule en matière plastique (1), et forment ainsi deux moitiés longitudinales égales (1a, 1b), dont les éléments de liaison (7) sont détachables au moyen d'un dispositif de séparation disposé dans l'organe de raccordement (2) qui est constitué de deux demi-organes de raccordement (2a, 2b) qui présentent des oreilles de manipulation (14) ou analogues, décalées l'une par rapport à l'autre, et forment entre eux une surface de déplacement (10) qui fait un angle aigu (α) avec la surface de séparation (11) définie par les deux rangées (8) précitées entre les deux moitiés longitudiales (1a, 1b) de la canule en matière plastique (1).

2. Cathéter intraveineux conforme à la revendiction 1, caractérisé en ce que les deux demi-organes de raccordement (2a, 2b) sont reliés l'un à l'autre par un pont séparable (9).

Fig. 1

Fig.2

Fig. 3